Europäisches Patentamt

European Patent Office

Office européen des brevets

(11) Veröffentlichungsnummer: **0 183 236**
A2

(12) **EUROPÄISCHE PATENTANMELDUNG**

(21) Anmeldenummer: 85115012.8

(22) Anmeldetag: 27.11.85

(51) Int. Cl.⁴: **A 61 B 17/22**

(30) Priorität: 28.11.84 DE 3443295

(43) Veröffentlichungstag der Anmeldung:
04.06.86 Patentblatt 86/23

(84) Benannte Vertragsstaaten:
AT BE CH DE FR GB IT LI LU NL SE

(71) Anmelder: Eisenmenger, Wolfgang Prof. Dr.
Landhausstrasse 7
D-7140 Ludwigsburg(DE)

(72) Erfinder: Eisenmenger, Wolfgang Prof. Dr.
Landhausstrasse 7
D-7140 Ludwigsburg(DE)

(54) Einrichtung zur berührungsfreien Zertrümmerung von Konkrementen im Körper von Lebewesen.

(57) Eine Einrichtung zur berührungsfreien Zertrümmerung von Konkrementen im Körper von Lebewesen, insbesondere zum Zertrümmern von Nierensteinen, umfaßt eine mit Stromstößen beaufschlagbare Spule, wobei durch diese Stromstöße in einer von der Spule durch eine Isolierschicht getrennten Metallmembran entgegengerichtete Ströme induziert werden, die zu einer Abstoßung der Metallmembran führen. Die Metallmembran steht mit einem Übertragungsmedium in Kontakt, an welches hierbei Stoßwellen abgegeben werden. Das Übertragungsmedium ist in einem durchgehenden kegelstumpfförmigen Hohlraum eines Bauteils, welches bevorzugt aus Material mit hohem Schallwellenwiderstand besteht, angeordnet. Durch den kegelstumpfförmigen Hohlraum wird die Ausbildung der Stoßfront günstig beeinflußt. Außerdem können die Anforderungen an die Impulsquelle reduziert werden, und gehäusebedingte Beugungs- und Reflexionserscheinungen sowie parasitäre Signale werden vermieden.

Fig. 1

Croydon Printing Company Ltd.

## Einrichtung zur berührungsfreien Zertrümmerung von Konkrementen im Körper von Lebewesen

Die Erfindung betrifft eine Einrichtung zur berührungsfreien Zertrümmerung von Konkrementen im Körper von Lebewesen, insbesondere zum Zertrümmern von Nierensteinen, mit einer kugelkalottenartig ausgebildeten Metallmembran, an deren nach außen gewölbter Fläche wenigstens eine von der Metallmembran durch eine Isolierschicht getrennte Spule angeordnet ist und deren nach innen gewölbte Fläche in Kontakt mit einem Übertragungsmedium steht, wobei die Spule mit Stromstößen beaufschlagbar ist, durch die die Metallmembran abgestoßen wird, welche dabei im wesentlichen auf das Konkrement fokussierte Stoßwellen in das Übertragungsmedium ausstrahlt.

Eine derartige Einrichtung ist aus der DE-OS 33 12 014 bekannt. Gegenüber anderen Vorrichtungen zur berührungsfreien Zertrümmerung von Konkrementen, die mit Unterwasserfunkenstrecken oder mit Ultraschall arbeiten und die in dieser DE-OS ausführlich besprochen sind, hat die dort beschriebene Einrichtung mehrere Vorteile, insbesondere einfachen Aufbau, hohe Lebensdauer und hohe Sicherheit sowie der Ausschluß von Gesundheitsrisiken für den Patienten, Möglichkeit des Verzichts auf ein Flüssigkeitsbad, leichtere Einstellbarkeit sowie bessere Reproduzierbarkeit.

Bei der Einrichtung gemäß der DE-OS 33 12 014 ist eine Leiteranordnung vorgesehen, die induktive Eigenschaften aufweist und als spiralförmige Spule oder als aus einem Flachband gewickelte Spule ausgebildet ist und die dort die Form einer Kugelkalotte hat. Bei Beaufschlagung dieser Spule mit einem Stromstoß, beispielsweise durch eine Kondensatorentladung über eine Funkenstrecke oder durch Beaufschlagung mit von einem Hochleistungspulsgenerator erzeugten Stromstößen werden in einer Metallmembran, die von der Spule durch eine dünne Isolierschicht getrennt ist, entgegengerichtete Ströme induziert, die zur Abstoßung dieser Membran führen. Da die Membran in Kontakt mit dem Übertragungsmedium steht, werden hierdurch Stoßwellen abgegeben, die aufgrund der kugelkalottenförmigen Ausbildung dieser Membran fokussiert werden.

Die vorliegende Erfindung hat sich die Aufgabe gestellt, eine Einrichtung der eingangs genannten und in der DE-OS 33 12 014 beschriebenen Art im Sinn einer besseren Fokussierung fortzubilden. Diese Aufgabe wird dadurch gelöst, daß auf der Abstrahlseite (d.h. auf der Seite der nach innen gewölbten Fläche) der Metallmembran ein Bauteil - vorzugsweise ein Gehäuseteil - angeordnet ist, das einen durchgehenden, kegelstumpfförmigen Hohlraum umschließt, dessen Öffnung von größerem Radius der Metallmembran und dessen Öffnung von kleinerem Radius dem zu zertrümmernden Konkrement zugewandt ist und in dem sich Übertragungsmedium befindet.

Durch diese Ausbildung des sich an die Metallmembran anschliessenden Bauteils wird erreicht, daß Beugungen und Reflexionen, wie sie an dem sich gemäß der DE-OS 33 12 014 an die Metallfolie anschließenden Gehäuseteil auftreten können und die zu Beeinträchtigungen der Ausbildung und der Fokussierung der Stoßwellen sowie zu unerwünschten parasitären Signalen führen können, vermieden werden. Es kann somit eine bessere Fokussierung sowie eine höhere Energiedichte im Brennpunkt erreicht werden, so daß der Wirkungsgrad der Einrichtung ohne aufwendige Maßnahmen verbessert werden kann.

Durch die kegelstumpfförmige Ausbildung des Hohlraums wird eine konzentrische und breitbandige Wellenausbreitung in Richtung auf den Fokus erzwungen. Dies führt einmal dazu, daß die für die Stoßwellenentwicklung erforderlichen Anfangsenergien, die durch die Kondensatorentladung oder durch den Hochleistungspulsgenerator bereitgestellt werden, reduziert werden. Die Einrichtung kann somit einfacher und kostengünstiger aufgebaut werden. Außerdem können nun an die Anfangssteilheiten der zu erzeugenden Druckimpulse geringere Anforderungen gestellt werden, d.h. die Druckimpulse können auch etwas weniger steil verlaufen, ohne daß die Wirkung beeinträchtigt wird. Auch dies führt dazu, daß die den Stromstoß erzeugende Impulsquelle einfacher aufgebaut sein kann. Schließlich wird durch die Erfindung auch die Ausbildung der für die Stoßwellenwirkung bei der Konkrementzertrümmerung wesentlichen Stoßfront günstig beeinflußt.

Eine weitere, ganz wesentliche Verbesserung der Fokussiereigenschaften ergibt sich, wenn das mit dem kegelstumpfförmigen Hohlraum versehene Bauteil wenigstens im Bereich des Hohlkegelstumpfs aus Material mit hohem Schallwellenwiderstand (hohem akustischem Wellenwiderstand) besteht, beispielsweise aus Metall und insbesondere rostfreiem Stahl. Hierdurch wird die Fokussierung weiter verbessert, da die Stoßwellen (diese Stoßwellen sind Kugelwellen) nunmehr in dem Hohlkegelstumpf "geführt" werden. Außerdem wird die Stoßwellenfront versteilert. Das mit dem kegelstumpfförmigen Hohlraum versehene Bauteil kann hierbei ganz aus Material mit hohem Schallwellenwiderstand bestehen; es genügt jedoch auch, wenn dieses Bauteil auf der dem Hohlraum zugewandten Flanke mit einer entsprechenden Auskleidung versehen ist.

Im Moment der Abgabe des Druckpulses von der Metallmembran an das Übertragungsmedium weist dieser Druckpuls noch keine steile Stoßfront auf. Diese Stoßfront bildet sich jedoch mit einer Dicke von etwa 1 Mikron während der Ausbreitung im Hohlkegel aus. Die Tiefe des Hohlraums, d.h. die Höhe des Hohlkegelstumpfs (Hohlkonus) muß so bemessen werden, daß sich über den durch diese Tiefe gegebenen Laufweg eine steile Stoßfront ausbilden kann. Ist der Druckpuls bei der Erzeugung steiler, so verkürzt sich der Laufweg und damit die erforderliche Tiefe des
Hohlraums. Außerdem kann die Dauer des Stoßwellenimpulses im

Fokus aufgrund der Beugungseigenschaften bei der sich an die
Ausbreitung im Hohlkegel anschließenden freien Ausbreitung
durch entsprechende Wahl des Öffnungswinkels des Hohlkegels
zusätzlich beeinflußt werden. Wird der Öffnungswinkel kleiner
gewählt, was auch für den Wirkungsgrad des elektromagnetischen
Stoßwellengenerators (Spule und Metallmembran sowie Einrichtung
zur Impulserzeugung) günstig ist, so sind kürzere Pulsdauern im
Fokus möglich. Dies wirkt sich auch bei der Zerkleinerung der
Konkremente in abgangsfähige Bruchstücke sehr vorteilhaft aus.

Vorteilhaft ist es, wenn das Übertragungsmedium ein Medium mit
stark nichtlinearem Kompressionsverhalten ist. Diese im Prinzip
bereits aus der DE-OS 33 12 014 bekannte Ausbildung hat im
Zusammenhang mit der vorliegenden Erfindung eine Verkürzung der
zur Ausbildung der Stoßfront erforderlichen Laufwege zur Folge,
so daß die Tiefe des Hohlkegelstumpfs bzw. Hohlkonus reduziert
werden kann. Als Übertragungsmedium findet bevorzugt eine
Flüssigkeit, insbesondere Halogenkohlenwasserstoffe oder ein
Material mit niedrigem Schubmodul, insbesondere ein gummielastischer Körper Verwendung.

In vorteilhafter Weiterbildung der Erfindung ist zwischen dem
Übertragungsmedium und dem Gewebe des Lebewesens eine Koppelanordnung aus einem Material mit niedrigem Schubmodul, insbesondere ein gummielastischer Koppelkörper oder eine Flüssigkeitskopplung, vorgesehen. In dieser Koppelanordnung sowie im
Gewebe liegt eine freie Stoßwellenausbreitung vor, nachdem sich
die Stoßfront bereits während der Ausbreitung in dem Hohlkegelstumpf ausgebildet hat. Die akustischen Eigenschaften der Koppelanordnung, beispielsweise Wellenwiderstand, Schallgeschwindigkeit und Formgebung, werden so gewählt, daß eine möglichst
optimale Übertragung der Stoßwelle auf das angrenzende Gewebe
erreicht wird.

**0183236**

Die Koppelanordnung hat jedoch noch weitere Vorteile. Insbesondere kann sie so ausgebildet sein, daß die Stoßwellen je nach Wunsch und Anwendungsfall zusätzlich fokussiert oder defokussiert werden. Auch eine Verschiebung des Fokusorts ist damit möglich. Die entsprechend ausgebildete Koppelanordnung hat in diesem Fall also die Rolle einer Feinfokussierung. Erreicht werden kann dies beispielsweise durch entsprechende äußere Formgebung der Koppelanordnung, durch Inhomogenitäten im Material dieser Koppelanordnung oder durch die Verwendung unterschiedlicher Materialien. Der angestrebte Effekt läßt sich besonders gut erreichen, wenn die Koppelanordnung auf ihrer dem Übertragungsmedium zugewandten Seite eine rotationshyperboloidartige Fläche aufweist. Diese Fläche kann jedoch auch so gestaltet werden, daß die am Übergang Koppelkörper/Gewebe auftretenden Fokussierungsfehler kompensiert werden.

Die zusätzliche Möglichkeit der Veränderung des Fokalabstandes ermöglicht über den entsprechenden Einfluß auf den Öffnungswinkel eine zusätzliche Variationsmöglichkeit der Pulsdauer im Fokus.

Weitere Merkmale und Vorteile ergeben sich aus den Unteransprüchen sowie aus der Beschreibung zur Zeichnung, in der einige bevorzugte Ausführungsbeispiele gezeigt sind, die im folgenden erläutert werden. Es zeigen

Fig. 1    einen schematischen Querschnitt durch eine erfindungsgemäße Einrichtung, bei der als Übertragungsmedium eine unter Druck stehende Flüssigkeit vorgesehen ist,

Fig. 2    eine der Fig. 1 entsprechende Darstellung einer weiteren Ausführungsform der Erfindung, bei der als Übertragungsmedium ein gummielastischer Körper dient und

Fig. 3    eine Ausführungsform mit einer Flüssigkeit als Übertragungsmedium und einem Koppelkörper, der zusätzlich fokussierende Eigenschaften aufweist.

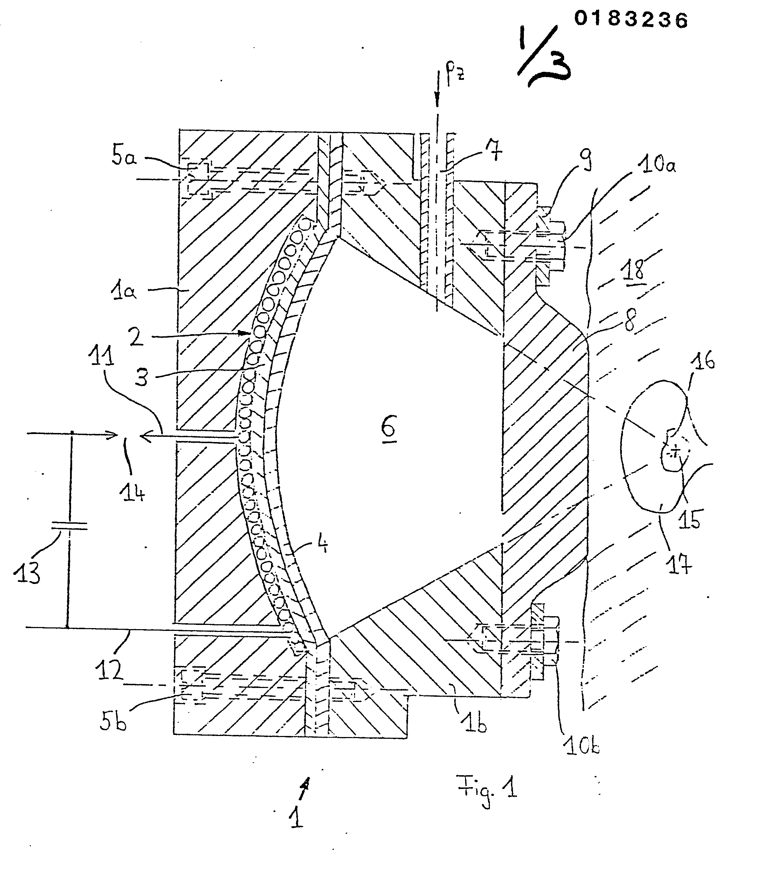

Das in der Fig. 1 gezeigte und im Ganzen mit 1 bezeichnete Gehäuse besteht aus zwei Gehäuseteilen 1a und 1b. In das Gehäuseteil 1a ist eine kugelkalottenförmige Spule 2 eingesetzt. Diese Spule besteht aus einem spiralförmig aufgewickelten Draht, beispielsweise Kupferdraht. An die Spule 2 schließt sich eine dünne Isolierschicht 3 an und an diese wiederum eine ebenfalls kugelkalottenförmige Metallmembran 4.

Die Isolierfolie 3 und die Metallmembran 4 sind zwischen den Gehäuseteilen 1a und 1b, welche durch Schrauben 5a und 5b verbunden sind, eingeklemmt.

In dem Gehäuseteil 1b befindet sich ein Hohlraum 6, der die Gestalt eines Kegelstumpfs oder Konus hat. Dieser Hohlraum ist mit einer Flüssigkeit, hier Wasser, gefüllt. Das Wasser steht unter statischem Überdruck, wobei ein Anschlußstutzen 7 vorgesehen ist, der an eine den Überdruck $p_z$ ausübende Druckquelle angeschlossen ist. Anstelle von Wasser kann vorteilhaft auch flüssiger Halogenkohlenwasserstoff, beispielsweise Tetrachlorkohlenstoff verwendet werden. Solche Medien besitzen ein starkes nichtlineares Kompressionsverhalten, d.h. diese Nichtlinearität ist größer als bei Wasser. Hierdurch wird der zur Ausbildung der Stoßfront erforderliche Laufweg verkürzt.

Auf der Seite der Öffnung von kleinerem Radius des in dem Gehäuseteil 1b vorgesehenen Hohlkegels 6 befindet sich ein Koppelkörper 8. Dieser Koppelkörper besteht im vorliegenden Fall aus gummielastischem Material, jedoch kann auch eine Flüssigkeitskopplung vorgesehen sein. Der Koppelkörper 8 ist über einen Befestigungsring 9 sowie Schrauben 10a und 10b mit dem Gehäuseteil 1b verschraubt.

Eine der innersten Windungen der Spule 2 sowie eine ihrer
äußersten Windungen sind über Leitungen 11 und 12 mit einer
Energiequelle verbunden, die impulsförmige Stromstöße abgeben
kann. Im gezeigten Ausführungsbeispiel ist hierfür ein Kondensator 13 sowie eine Funkenstrecke 14 vorgesehen, jedoch können
auch andere Anordnungen, wie beispielsweise ein gesteuerter
Hochleistungspulsgenerator, eingesetzt werden. Der im
Augenblick der Impulsabgabe durch die Spule 2 fließende Strom
induziert in der Metallmembran 4 entgegengesetzte Ströme, die
zur Abstoßung der Metallmembran führen, so daß letztere an das
Übertragungsmedium im konischen Hohlraum 6 eine Stoßwelle abgibt. Diese Stoßwelle ist aufgrund der kugelkalottenförmigen
Ausführung der Metallmembran sowie der konusartigen Gestaltung
des Hohlraums 6 im Gehäuseteil 1b auf einen Fokalpunkt 15 fokussiert, der in einem Konkrement 16, das hier ein Nierenstein
in einer Niere 17 ist, liegt.

Durch die hohlkegelstumpfförmige Ausbildung des Hohlraums 6
wird eine bessere Fokussierung der entstehenden Stoßwellen erreicht und zugleich die Stoßfront günstig beeinflußt. Gehäusebedingte Beugungs- und Reflexionserscheinungen sowie parasitäre
Signale können nicht mehr auftreten. Diese Effekte werden noch
dadurch verstärkt, daß das Gehäuseteil 1b aus einem Material
mit hohem Schallwellenwiderstand, hier rostfreiem Stahl, ausgeführt ist. Aufgrund der günstigen Ausbildung der Stoßfront können nun auch die Anfangsenergien der Kondensatorentladung sowie
die Anfangssteilheiten der erzeugten Druckimpulse reduziert
werden.

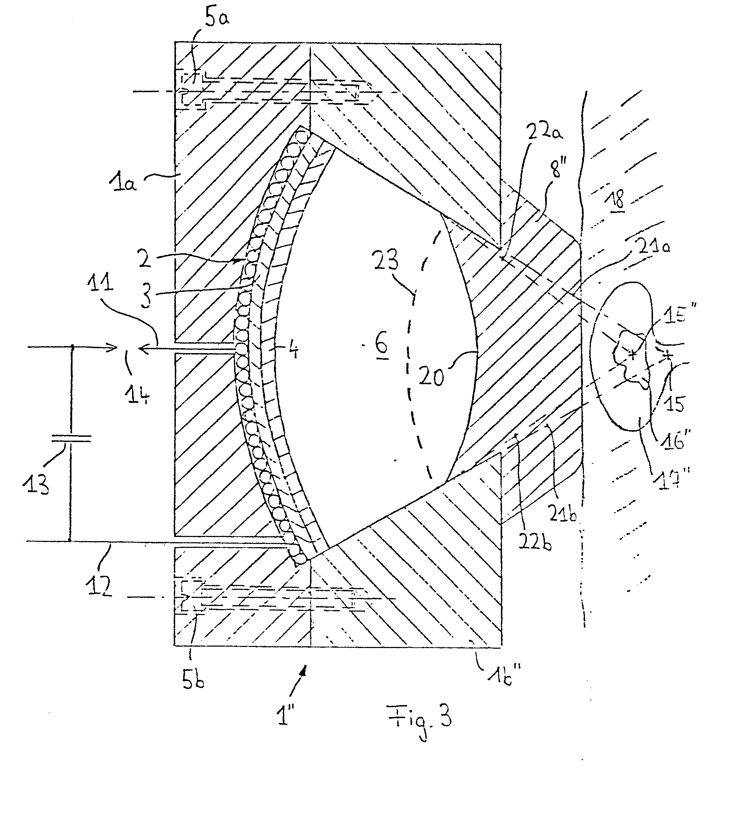

Der Koppelkörper 8 liegt hierbei direkt am Gewebe 18 des Lebewesens an. Die Stoßwellenübertragung erfolgt über das Übertragungsmedium 6 sowie den Koppelkörper 8. Es ist natürlich aber
auch möglich, das Übertragungsmedium sowie den Koppelkörper aus
demselben Material herzustellen. Eine Verschiebung des Fokalpunktes kann erreicht werden, wenn der Krümmungskreis der kugelkalottenförmigen Metallmembran 4 verstellbar ausgeführt

wird. Hier sind jedoch noch weitere, anhand der Fig. 3 zu diskutierende und technisch einfachere Möglichkeiten gegeben.

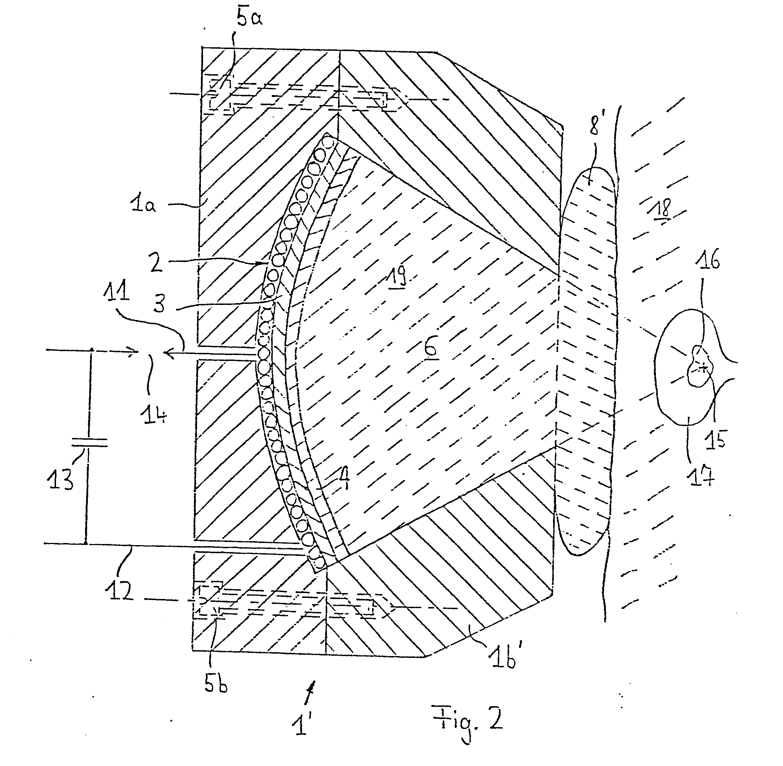

Bei dem in der Fig. 2 gezeigten zweiten Ausführungsbeispiel
werden für identische Teile dieselben Bezugszeichen verwendet
wie in der Fig. 1; einander entsprechende Bauteile sind durch
einen Strich gekennzeichnet. Das Übertragungsmedium ist hier
ein gummielastischer Körper 19, der in dem konusförmigen Hohlraum 6 angeordnet ist. Dieser gummielastische Körper besitzt
ebenso wie die Flüssigkeitsfüllung im Ausführungsbeispiel der
Fig. 1 nichtlineares Kompressionsverhalten, wodurch sich die
für die Ausbildung der Stoßfront erforderlichen Laufwege verkürzen, und steht, ebenso wie diese, unter statischem Überdruck. Der gummielastische Körper 19 kann hier allerdings in
den Hohlraum 6 eingepreßt werden, so daß eine Druckzuführung
von außen nicht erforderlich ist. An diesen Körper 19 schließt
sich ein Koppelkörper 8' an, der, wie gezeigt, mit dem Übertragungsmedium 19 in Kontakt steht und mit diesem auch verbunden sein kann. Das Übertragungsmedium und der Koppelkörper
können aus unterschiedlichem oder auch aus demselben Material
bestehen. Beim Ausführungsbeispiel gemäß Fig. 2 sind die Isolierschicht 3 und die Metallmembran 4 lediglich in das Gehäuseteil 1a eingesetzt und nicht zwischen den Gehäuseteilen 1a und
1b eingeklemmt.

Im Ausführungsbeispiel gemäß Fig. 3 sind die Bezugszeichen, die
sich lediglich auf entsprechende, nicht aber auf identische
Bauteile beziehen, mit zwei Strichen bezeichnet. An diesem Beispiel soll die Möglichkeit einer Veränderung des Fokalabstandes
ohne Veränderung der äußeren Form der Metallmembran gezeigt
werden.

Als Übertragungsmedium in dem Hohlraum 6 dient hier wieder
unter Überdruck stehendes Wasser. Die Druckzuführung ist hier
nicht gezeigt. Der Koppelkörper 8" ist hier jedoch anders ausgeführt und ragt zum Teil in den von dem Gehäuseteil 1b" gebil-

deten Hohlraum, wodurch zugleich ein guter Verschluß erreicht wird. Seine nach innen weisende Oberfläche 20 besitzt die Form eines Rotationshyperboloids einfacher oder höherer Ordnung. Dadurch läßt sich erreichen - hier durch äußere Formgebung - daß der Fokalabstand verändert wird. Natürlich kann dieser Effekt aber auch dadurch erreicht werden, daß der Koppelkörper 8" aus verschiedenen Materialien zusammengesetzt ist oder daß der Koppelkörper 8" inhomogen aufgebaut ist.

Ohne die spezielle Ausbildung des Koppelkörpers 8" würden die Stoßwellen, wie durch die Linien 21a und 21b angedeutet, auf den Fokalpunkt 15 fokussiert werden. Durch die spezielle Formgebung des Koppelkörpers 8" kann nun jedoch erreicht werden, daß der Fokuspunkt, wie durch die Linien 22a und 22b angedeutet, in den neuen Fokalort 15", an dem sich das Konkrement 16", hier ein Nierenstein einer Niere 17", befindet, verschoben wird. Hierdurch kann der Fokusort den jeweiligen Gegebenheiten angepaßt werden. Andere Möglichkeiten zur Variation des Fokalorts bestehen darin, daß der Krümmungskreis der Metallmembran 4 verstellt wird und/oder daß der Öffnungswinkel des Hohlkonus 6 abgeändert wird.

Statt der hyperbolischen Ausbildung der Oberfläche 20 kann auch, wie gestrichelt mit dem Bezugszeichen 23 angedeutet, eine zur Membran 4 konzentrische Kugelkalottenoberfläche vorgesehen werden, die den Koppelkörper 8" zu dem Übertragungsmedium hin begrenzt. Dadurch tritt beim Übergang Übertragungsmedium/Koppelkörper aufgrund des senkrechten Welleneinfalls keine Brechung auf.

Anmelder:

Prof. Dr. Wolfgang Eisenmenger
Landhausstrasse 7

7140 Ludwigsburg

Ansprüche

1.    Einrichtung zur berührungsfreien Zertrümmerung von Konkrementen in Körpern von Lebewesen, insbesondere zum Zertrümmern von Nierensteinen, mit einer kugelkalottenartig ausgebildeten Metallmembran, an deren nach außen gewölbter Fläche wenigstens eine von der Metallmembran durch eine Isolierschicht getrennte Spule angeordnet ist und deren nach innen gewölbte Fläche in Kontakt mit einem Übertragungsmedium steht, wobei die Spule mit Stromstößen beaufschlagbar ist, durch die die Metallmembran abgestoßen wird, welche dabei im wesentlichen auf das Konkrement fokussierte Stoßwellen in das Übertragungsmedium ausstrahlt, dadurch gekennzeichnet, daß auf der Abstrahlseite der Metallmembran (4) ein Bauteil - vorzugsweise ein Gehäuseteil (1b, 1b', 1 b") - angeordnet ist, das einen durchgehenden kegelstumpfförmigen Hohlraum (6) umschließt, dessen Öffnung von größerem Radius der Metallmembran (4) und dessen Öffnung von kleinerem Radius dem zu zertrümmernden Konkrement (16, 16") zugewandt ist und in dem sich Übertragungsmedium befindet.

**0183236**

2. Einrichtung nach Anspruch 1, dadurch gekennzeichnet, daß das Bauteil wenigstens im Bereich des Hohlkegelstumpfs aus Material mit hohem Schallwellenwiderstand besteht.

3. Einrichtung nach Anspruch 2, dadurch gekennzeichnet, daß das Material mit hohem Schallwellenwiderstand Metall, insbesondere rostfreier Stahl, ist.

4. Einrichtung nach einem oder mehreren der vorhergehenden Ansprüche, dadurch gekennzeichnet, daß das Übertragungsmedium ein unter statischem Druck stehendes Medium mit nichtlinearem Kompressionsverhalten ist.

5. Einrichtung nach Anspruch 4, dadurch gekennzeichnet, daß das Übertragungsmedium eine Flüssigkeit, insbesondere Wasser, ist.

6. Einrichtung nach Anspruch 4, dadurch gekennzeichnet, daß als Übertragungsmedium Material mit niedrigem Schubmodul, beispielsweise ein gummielastischer Körper (19), vorgesehen ist.

7. Einrichtung nach einem oder mehreren der vorhergehenden Ansprüche, dadurch gekennzeichnet, daß zwischen dem Übertragungsmedium und dem Gewebe (18) des Lebewesens eine Koppelanordnung aus einem Material mit niedrigem Schubmodul, vorzugsweise eine gummielastischer Koppelkörper (8, 8', 8") oder eine Flüssigkeitskopplung, vorgesehen ist.

8. Einrichtung nach Anspruch 7, dadurch gekennzeichnet, daß die Koppelanordnung, insbesondere durch äußere Formgebung, so ausgebildet ist, daß die Stoßwellen zusätzlich fokussiert oder defokussiert werden.

9. Einrichtung nach Anspruch 8, dadurch gekennzeichnet, daß die Koppelanordnung auf ihrer dem Übertragungsmedium zuge-

wandten Seite eine rotationshyperboloidartige Fläche (20) aufweist.

10. Einrichtung nach Anspruch 7, dadurch gekennzeichnet,
daß die Koppelanordnung auf ihrer dem Übertragungsmedium zugewandten Seite kugelkalottenförmig und konzentrisch zu der Metallmembran (4) ausgebildet ist.

11. Einrichtung nach wenigstens einem der Ansprüche 7 bis
10, dadurch gekennzeichnet, daß die Koppelanordnung wenigstens
teilweise in dem kegelstumpfförmigen Hohlraum des Bauteils angeordnet ist.

Fig. 1

0183236

Fig. 2

3/3

0183236

Fig. 3